(19) Europäisches Patentamt
European Patent Office
Office européen des brevets

(11) Veröffentlichungsnummer: **0 025 907**
**B1**

(12) # EUROPÄISCHE PATENTSCHRIFT

(45) Veröffentlichungstag der Patentschrift: **20.06.84**

(51) Int. Cl.³: **C 07 C 119/042,** C 07 C 119/045, C 07 C 118/00

(21) Anmeldenummer: **80105232.5**

(22) Anmeldetag: **03.09.80**

(54) **Halogenierte, tertiäre Alkylisocyanate und Verfahren zu ihrer Herstellung.**

(30) Priorität: **13.09.79 DE 2937062**

(43) Veröffentlichungstag der Anmeldung: **01.04.81 Patentblatt 81/13**

(45) Bekanntmachung des Hinweises auf die Patenterteilung: **20.06.84 Patentblatt 84/25**

(84) Benannte Vertragsstaaten: **BE CH DE FR GB LI NL**

(56) Entgegenhaltungen:
**DE - A - 1 418 666**
**DE - B - 1 090 197**
**DE - B - 1 122 058**
**US - A - 3 277 140**
**US - A - 3 535 360**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Koenig, Karl-Heinz, Dr., Pierstrasse 8A, D-6710 Frankenthal (DE)**
Erfinder: **Schwendemann, Volker, Dr., Hauptstrasse 64A, D-6901 Wiesenbach (DE)**
Erfinder: **Feuerherd, Karl-Heinz, Dr., Berner Weg 34, D-6700 Ludwigshafen (DE)**

**EP 0 025 907 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die Erfindung betrifft halogenierte, tertiäre Alkylisocyanate und Verfahren zu ihrer Herstellung durch Umsetzung von tertiären Alkylisocyanaten mit Halogen, Sulfurylchlorid und/oder Sulfurylbromid.

Die Halogenierung von aliphatischen Isocyanaten mit mindestens einem substituierbaren Wasserstoffatom am α-ständigen Kohlenstoff zum Stickstoff der Isocyanatgruppe ist bekannt (DE-PS 1 122 058, Angew. Chemie, Band 74, S. 848 bis 855 (1962) und Band 80, Seiten 942 bis 953 [1968]). In DE-PS 1 122 058 wird darauf hingewiesen, dass Halogenalkylisocyanate sehr reaktionsfähige Substanzen sind (2. Spalte, Zeilen 33 und 34) und α-Halogenalkylisocyanate zur Kondensation oder Polymerisation neigen (2. Spalte, Zeilen 30 und 31); Beispiel 3 zeigt neben einer 68%igen Ausbeute grössere Destillationsrückstände.

Ohne Zusatz von Lösungsmitteln treten bei der Halogenierung von aliphatischen Isocyanaten durch intramolekulare Kondensation oft Verharzung und schlechte Ausbeuten auf, wie die Angew. Chemie (loc. cit., Seite 946) lehrt. Ebenfalls zeigt diese Literaturstelle, dass durch Anlagerung des bei der Reaktion gebildeten Chlorwasserstoffs an das Isocyanat grosse Mengen an dem entsprechenden Carbamidsäurechlorid gebildet werden, und empfiehlt zur Vermeidung dieser Nebenreaktion, dass als Ausgangsstoffe Carbamidsäurechloride verwendet werden.

Führt man das in DE-PS 1 122 058 beschriebene Verfahren der α-Halogenierung aliphatischer Isocyanate im industriellen Massstab durch, so zeigt sich, dass bei der Aufdestillation des anfallenden Gemisches von halogenierten Isocyanaten verschiedenen Halogenierungsgrades erhebliche, nicht destillierbare Rückstände von Polymeren oder Polykondensaten verbleiben, im allgemeinen ca. 20 bis 40 Gew.-%, bezogen auf das halogenierte Produkt.

Die in der DE-A-1 418 666 beschriebenen hoch halogenierten Isocyanate enthalten in α-Position zur Isocyanatfunktion weitere Halogenatome. Diese α-Halogenatome geben bei der Umsetzung dieser Isocyanate stets zu Nebenreaktionen Anlass, da diese α-halogenierten Isocyanate in einem Chlorotropie-Gleichgewicht mit ihrer Chlorcarbonyliminform stehen (H. Holtschmidt, Angew. Chem. 74, 848 (1962).

R-CHCl-NCO ⇌ R-CH=N-COCl

Diese Ethylidencarbamoylchloride sind wegen ihrer labilen CN-Doppelbindung für selektive Reaktionen weniger geeignet. So lehrt Journal für praktische Chemie 320, 627, Abschnitt 2 (1978), dass α-halogenierte Isocyanate mit Aminen z.B. ein Gemisch mehrerer Additionsprodukte ergeben.

Es wurde nun gefunden, dass man halogenierte, tertiäre Alkylisocyanate der Formel

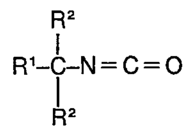

I,

worin $R^1$ den Rest $R^2$ oder den Rest

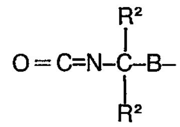

bedeutet, die einzelnen Reste $R^2$ gleich oder verschieden sind und jeweils einen halogenierten oder halogenfreien Alkylrest mit 1 bis 6 Kohlenstoffatomen bedeuten, jeweils zwei über ein Kohlenstoffatom benachbarte Reste $R^2$ auch Glieder eines halogenfreien oder halogenierten, alicyclischen, 5- oder 6gliedrigen Ringes bilden können, B einen halogenfreien oder halogenierten Alkylenrest mit 1 bis 6 Kohlenstoffatomen bezeichnet, mindestens einer der Reste $R^2$ ein oder mehrere Halogenatome trägt, wobei die Gesamtzahl der Halogenatome mindestens 3 und, wenn $R^1$ den Rest $R^2$ bezeichnet und alle 3 Reste $R^2$ zusammen 3 Kohlenstoffatome enthalten, mindestens 4 beträgt, alle Reste $R^2$ und gegebenenfalls B zusammen höchstens 9 Kohlenstoffatome bezeichnen, durch Halogenierung von Isocyanaten oder Carbamidsäurechloriden, vorteilhaft erhält, wenn man tertiäre Alkylisocyanate der Formel

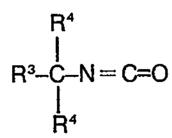

II,

oder tertiäre Carbamidsäurehalogenide der Formel

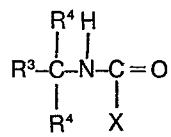

III,

worin $R^3$ den Rest $R^4$ oder den Rest

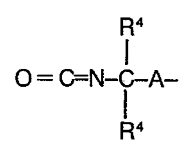

oder den Rest

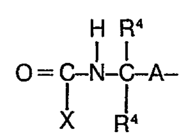

bedeutet, die einzelnen Reste $R^4$ gleich oder verschieden sein können und jeweils einen halogenierten oder halogenfreien Alkylrest mit 1 bis 6 Kohlenstoffatomen bedeuten, jeweils 2 über ein Kohlenstoffatom benachbarte Reste $R^4$ auch Glieder eines halogenfreien oder halogenierten, alicyclischen, 5- oder 6gliedrigen Ringes bilden können, A einen Alkylenrest oder Halogenalkylenrest mit 1 bis 6 Kohlenstoffatomen bezeichnet, alle Reste $R^4$ und gegebenenfalls A zusammen höchstehs 9 Kohlenstoffatome bezeichnen, X für ein

Halogenatom steht, mit Halogen, Sulfurylchlorid und/oder Sulfurylbromid umsetzt.

Weiterhin wurden die neuen halogenierten, tertiären Alkylisocyanate der Formel

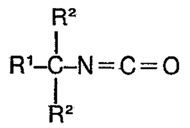

I,

worin $R^1$ den Rest $R^2$ oder den Rest

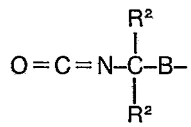

bedeutet, die einzelnen Reste $R^2$ gleich oder verschieden sind und jeweils einen halogenierten oder halogenfreien Alkylrest mit 1 bis 6 Kohlenstoffatomen bedeuten, jeweils zwei über ein Kohlenstoffatom benachbarte Reste $R^2$ auch Glieder eines halogenfreien oder halogenierten, alicyclischen, 5- oder 6gliedrigen Ringes bilden können, B einen halogenfreien oder halogenierten Alkylenrest mit 1 bis 6 Kohlenstoffatomen bezeichnet, mindestens einer der Reste $R^2$ ein mehrere Halogenatome trägt, wobei die Gesamtzahl der Halogenatome mindestens 3 und, wenn $R^1$ den Rest $R^2$ bezeichnet und alle 3 Reste $R^2$ zusammen 3 Kohlenstoffatome enthalten, mindestens 4 beträgt, alle Reste $R^2$ und gegebenenfalls B zusammen höchstens 9 Kohlenstoffatome bezeichnen, gefunden.

Die Umsetzung wird für den Fall der Verwendung von tertiärem Butylisocyanat und Chlor durch die folgenden Formeln wiedergegeben:

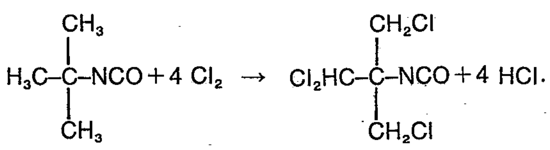

Im Hinblick auf den Stand der Technik liefert das Verfahren nach der Erfindung überraschend auf einfachem und wirtschaftlichem Wege halogenierte, tertiäre Alkylisocyanate in guter Ausbeute und Reinheit. Es war überraschend, dass gerade Alkylisocyanate ohne α-ständigem Wasserstoffatom mit so vorteilhaften Ergebnissen umgesetzt werden und keine oder nur geringfügige Destillationsrückstände ergeben.

Bevorzugte Ausgangsstoffe II und III und demzufolge bevorzugte Endstoffe I sind solche, in deren Formeln $R^1$ den Rest $R^2$ oder den Rest

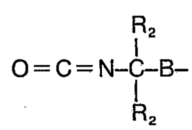

bedeutet, die einzelnen Reste $R^2$ gleich oder verschieden sind und jeweils einen halogenfreien oder halogenierten Alkylrest mit 1 oder 2 Kohlenstoffatomen bedeuten, jeweils zwei über ein Kohlenstoffatom benachbarte Reste $R^2$ zusammen mit diesem Kohlenstoffatom auch Glieder eines halogenfreien oder halogenierten, alicyclischen, 5- oder 6gliedrigen Ringes bilden können, $R^3$ den Rest $R^4$- oder den Rest

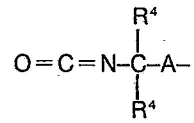

oder den Rest

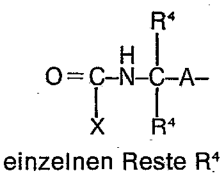

bedeutet, die einzelnen Reste $R^4$ gleich oder verschieden sein können und jeweils einen Alkylrest mit ein oder 2 Kohlenstoffatomen bedeuten, jeweils 2 über ein Kohlenstoffatome benachbarte Reste $R^4$ zusammen mit diesem Kohlenstoffatom auch Glieder eines alicyclischen, 5- oder 6gliedrigen Ringes bilden können, A einen Alkylenrest oder Halogenalkylenrest mit 0, 1 oder 2 Halogenatomen und ein bis 2 Kohlenstoffatomen bezeichnet, B einen halogenfreien oder halogenierten Alkylrest mit 0, 1 oder 2 Halogenatomen und 1 bis 2 Kohlenstoffatomen bedeutet, mindestens einer der Reste $R^2$ ein oder mehrere Halogenatome trägt, X für ein Bromatom oder insbesondere ein Chloratom steht und jedes Halogenatom im Endstoff I ein Bromatom oder insbesondere ein Chloratom bezeichnet.

Beispielsweise sind folgende Monoisocyanate II und Monocarbamidsäurehalogenide III geeignet: Trimethyl-, Triäthyl-, Tripropyl-, Triisopropyl-, Tributyl-, Triisobutyl-, Tri-sek.-butyl-, Tri-tert.-butyl-, Tripentyl-, Triisopentyl-, Trihexyl-methylisocyanat; 1,1-Dimethylpropylisocyanat, 1,1-Dimethylbutylisocyanat, 1,1-Dimethylpentylisocyanat, 1,1-Dimethylhexylisocyanat, 1,1-Dimethylheptlyisocyanat und entsprechende 1-Äthyl-1-methyl-, 1-Propyl-1-methyl-, 1- Butyl-1-methyl-, 1-Pentyl-1-methyl-, 1-Hexyl-1-methyl-, 1-Heptyl-1-methyl-, 1,1-Diäthyl-, 1-Äthyl-1-propyl-, 1-Äthyl-1-butyl-, 1-Äthyl-1-pentyl-, 1-Äthyl-1-hexyl-, 1-Äthyl-1-isobutyl-Homologe; 1-Propyl-Cyclohexyl-(1)-isocyanat, 1-Butyl-Cyclohexyl-(1)-isocyanat, 1-Methyl-Cyclohexyl-(1)-isocyanat, 1-Äthyl-cyclohexyl-(1)-isocyanat, 1-Methylcyclopentyl-(1)-isocyanat, 1-Äthylcyclopentyl-(1)-isocyanat; entsprechende substituierte Carbamidsäurechloride und Carbamidsäurebromide; bevorzugt sind tert.-Butylisocyanat, tert.-Amylisocyanat, 1,1-Dimethylbutylisocyanat, 1,1-Dimethylheptylisocyanat, 1,1-Diäthyl-propylisocyanat, 1-Methyl-1-äthylpropylisocyanat, Triäthylmethylisocyanat.

Als tertiäre Diisocyanate II und Dicarbamidsäurehalogenide III lassen sich nach dem erfindungsgemässen Verfahren z.B. verwenden:
1,1,3,3-Tetramethylpropan-1,3-diisocyanat,
1,1,3,3-Tetraäthylpropan-1,3-diisocyanat,
1,1,3,3-Tetraisopropylpropan-1,3-diisocyanat,
1,1,3,3-Tetrapropylpropan-1,3-diisocyanat,
1,1,3,3-Tetrabutylpropan-1,3-diisocyanat,
1,1,3,3-Tetraisobutylpropan-1,3-diisocyanat,
1,1,3,3-Tetra-sek.-butylpropan-1,3-diisocyanat,
1,1,3,3-Tetra-tert.-butylpropan-1,3-diisocyanat,

1,1,3,3-Tetrapentylpropan-1,3-diisocyanat,
1,1,3,3-Tetrahexylpropan-1,3-diisocyanat
und entsprechend in 1,1-Stellung und ω,ω-Stellung substituierte Butan-1,4-, Pentan-1,5-, Hexan-1,6-, Heptan-1,7-, Octan-1,8-diisocyanate; homologe durch vorgenannte Substituenten Methyl-, Äthyl-, Propyl-, Isopropyl-, Butyl-, Isobutyl-, sek.-Butyl-, tert.-Butyl-, Pentyl-, Isopentyl-, Hexylgruppe in den 4,1,1- und ω,ω-Stellungen unterschiedlich substituierte Diisocyanate, wobei 2 oder 3 Substituenten untereinander gleich sein können;
1,2-Dicyclohexyl-(1′-1″)-äthan-1′,1″-diisocyanat,
Dicyclohexyl-(1′,1″)-methan-1′,1″-diisocyanat,
1,3-Dicyclohexyl-(1′,1″)-propan-1′,1″-diisocyanat
1,4-Dicyclohexyl-(1′,1″-butan-1′,1″-diisocyanat
und entsprechende Dicyclopentylverbindungen; entsprechende Dicarbamidsäurechloride, Dicarbamidsäurebromide und
Monoisocyanato-mono-carbamidsäurechloride,
Monoisocyanato-monocarbamidsäure-bromide;
bevorzugt sind
1,1,4,4(Tetramethyl)butan-1,4-diisocyanat,
1,1,6,6(Tetramethyl)-hexan-1,6-diisocyanat,
1,4-Dimethyl-1,4-di(isobutyl)-butan-1,4-diisocyanat,
1,6-Dimethyl-1,6-di(isohexyl)hexan-1,6-diisocyanat,
1,4-Dimethyl-1,4-di(isopropyl)butan-1,4-diisocyanat,
1,6-Dimethyl-1,6-di(isopropyl)hexan-1,6-diisocyanat,
1,4-Dimethyl-1,4-di = (äthyl)butan-1,4-diisocyanat,
1,6-Dimethyl-1,6-di(äthyl)hexan-1,6-diisocyanat,
1,4-(Dicyclohexyl)-(1′,1″)-butan-1′,1″-diisocyanat,
1,6-(Dicyclohexyl)-(1′,1″)-hexan-1′,1″-diisocyanat.

Man setzt die Ausgangsstoffe II oder III mit Halogen, Sulfurylchlorid oder Sulfurylbromid als Halogenierungsmittel, bevorzugt mit Jod, Brom und insbesondere mit Chlor, um. Im allgemeinen erhält man bei der Halogenierung Gemische halogenierter Endstoffe I mit unterschiedlicher Zahl der Halogensubstituenten. Zuerst werden bevorzugt die β-ständigen Kohlenstoffatome halogeniert. Die Hauptkomponente an den Endstoffen I im Gemisch trägt in der Regel eine dem Molverhältnis von Ausgangsstoff und Halogenierungsmittel entsprechende Zahl der Halogensubstituenten am Molekül.

Die Ausgangsstoffe II oder III können in stöchiometrischer Menge verwendet werden, z.B. zur Herstellung von Trichlor-tert.-butylisocyanat 3 Mol Chlor je Mol tert.-Butylisocyanat. Bevorzugt wird man für die Herstellung von disubstituierten, trisubstituierten und höher halogenierten Alkylisocyanaten einen Überschuss an Halogenierungsmittel, bezogen auf Ausgangsstoff II oder III, verwenden; es kommen Mengen über der stöchiometrischen Menge bis zu 15 Mol, zweckmässig von 0,5 bis 8 Mol Halogenierungsmittel, bezogen auf Mol Ausgangsstoff II oder III, in Betracht. Bei der Herstellung von weitgehend monosubstituierten Verbindungen wird vorteilhaft ein Unterschuss an Chlor (Anchlorierung) verwendet, es kommen Mengen unterhalb der stöchiometrischen Menge bis zu 0,95 Mol, zweckmässig von 0,6 bis 0,9 Mol Halogenierungsmittel in Frage. Bevorzugt wird für höherhalogenierte Produkte eine Kreislaufhalogenierung verwendet, bei der nicht verbrauchtes elementares Halogen wieder zurückgeführt werden kann. Im übrigen kann der Halogenüberschuss umso geringer sein, je schneller das Halogen umgesetzt wird. Bei der Verwendung von Sulfurylhalogeniden setzt man zweckmässig einen Halogenierungskatalysator wie Benzoylperoxid, Azodiisobutyronitril oder Ascaridol in katalytischen Mengen, zweckmässig von 0,01 bis 0,1 Gew.-%, bezogen auf das Halogenierungsmittel, zu.

Die Reaktion wird bevorzugt unter Belichtung durchgeführt, wobei für die Erzeugung des Lichtes vorzugsweise alle im sichtbaren Bereich strahlenden Lichtquellen verwendet werden können. So kann z.B. mit Sonnenlicht oder künstlichem Licht, z.B. von Quarzbrennern, Quecksilberdampflampen, Tageslichtlampen, Leuchtstoffröhren, belichtet werden. Vorteilhaft verwendet man Tauchlampen, die vom Reaktionsgemisch umspült werden, insbesondere im Falle von Kreislaufapparaten in einem der Vertikalrohre. Bevorzugt sind Lichtquellen mit einem hohen Strahlungsanteil im Bereich der Wellenlängen von 3000 bis 5000 Angström.

Die Halogenierungstemperatur richtet sich vorteilhaft nach dem Halogenierungsgrad des Endstoffs I. So ist es bei niederen gewünschten Halogenierungsgraden, z.B. der Substituierung der Ausgangsstoffe II und III mit 1 bis 2 Halogenatomen, zweckmässig, zunächst bei einer Temperatur von 0 bis 10 °C zu beginnen und die Temperatur langsam bis ca. 50 °C zu steigern. Bei höheren erwünschten Halogenierungsgraden, z.B. die Substituierung der Ausgangsstoffe II und III mit 3 bis 6 Halogenatomen oder bei Verwendung des Ausgangsstoffs III beginnt man zur schnelleren Halogenaufnahme schon bei Temperaturen von 50 bis 100 °C und steigert die Temperatur rascher. Mit fortschreitendem Halogenierungsgrad ist es zur Aufrechterhaltung einer hinreichenden Halogenaufnahme vorteilhaft, die Reaktionstemperatur bis maximal 230 °C, bevorzugt 160 bis 180 °C zu steigern. In der Regel wird die Umsetzung bei einer Temperatur von −10 bis 230 °C, vorzugsweise 40 bis 180 °C, insbesondere 60 bis 130 °C, drucklos oder unter Druck, kontinuierlich oder diskontinuierlich durchgeführt. Bevorzugt wird in Abwesenheit zusätzlicher Lösungsmittel umgesetzt.

Die Reaktion kann wie folgt durchgeführt werden:
Der Ausgangsstoff II bzw. III wird bei der Reaktionstemperatur mit dem Halogenierungsmittel während 2 bis 50 Std. umgesetzt, wobei im Falle von elementarem Halogen dieses langsam und portionsweise während der Reaktionszeit zugesetzt wird. Zweckmässig wird in vorgenannter Weise die Temperatur innerhalb des vorgenannten Temperaturintervalls während der Reaktion erhöht. Aus dem Reaktionsgemisch wird im Falle eines einzelnen Hauptprodukts der Endstoff in üblicher Weise, z.B. durch fraktionierte Destillation,

abgetrennt. In der Mehrzahl der Fälle, gerade im industriellen Betrieb, handelt es sich um Gemische von Endstoffen, die durch Destillation gereinigt werden können, meistens aber der Weiterverarbeitung, z.B. der Herstellung von Appretiermitteln, z.B. Flammschutzmitteln, direkt zugeführt werden. Die niederhalogenierten Isocyanate des Destillationsvorlaufs kann man wieder als Vorprodukte für die Synthese höherhalogenierter Fraktionen eines nächsten Ansatzes verwenden. Die maximale Halogenaufnahme wird bei etwa 80 bis 85% der Theorie erreicht. Die Ausgangsstoffe II sind vorteilhafter als die Ausgangsstoffe III, da sie in kürzerer Zeit umgesetzt werden können.

Die nach dem Verfahren der Erfindung herstellbaren, halogenierten, tertiären Alkylisocyanate sind wertvolle Ausgangsstoffe für die Herstellung von Schädlingsbekämpfungsmitteln, Lederfettungsmitteln, Pharmaceutica, Flammschutzmitteln, Schmierölen, Kunstharzen, Gleitmitteln. So lassen sich die chlorierten Monoisocyanate zum Endgruppenverschluss von hydroxylgruppenhaltigen Polymeren und die mehrfach chlorierten Diisocyanate direkt als bifunktionelle Synthesebausteine für flammfeste Polymere, z.B. Polyurethane, verwenden. Bezüglich der Verwendung wird auf vorgenannte Veröffentlichungen verwiesen.

Die in den folgenden Beispielen aufgeführten Teile bedeuten Gewichtsteile.

Beispiel 1

In 90 Teile tert.-Butylisocyanat wird unter Bestrahlung mit einer Leuchtstoffröhre (4 000 bis 7 000 Angström) bei 2 °C Chlor eingeleitet. Während der Chloraufnahme steigert man die Temperatur während 3 Stunden allmählich bis auf 95 °C. Nachdem 126 Teile Chlor aufgenommen werden, wird das Gemisch fraktioniert destilliert. Die Chlorabsorption entspricht etwa der Substitution von 4 Wasserstoffatomen. Man erhält 48 Teile eines Vorlaufs niederhalogenierter Isocyanate, die bei einem erneuten Chlorierungsansatz wieder eingesetzt werden (Kp. 27 mbar, 100 bis 116 °C), 70 Teile Tetrachlor-6-butylisocyanat (Kp. 27 mbar, 119 bis 130 °C) und 65 Teile eines Gemisches von Pentachlor-t-butylisocyanat und Hexachlor-t-butylisocyanat vom Kp 0,13 mbar, 60 bis 73 °C.

Beispiel 2

In 40 Teile 1,1,4,4-Tetramethylbutan-1,4-diisocyanat leitet man bei 50 °C unter Belichtung mit einer Leuchtstoffröhre (4 000 bis 7 000 Angström) elementares Chlor ein, wobei man die Einleitungstemperatur während 6 Stunden langsam auf 180 °C steigert. 62 Teile Chlor werden aufgenommen. Die Chlorabsorption entspricht etwa der Substitution von 8 Wasserstoffatomen. Das ölige Produkt wird fraktioniert destilliert. Man erhält folgende Fraktionen:

a) Kp (0,27 mbar) 54 bis 108 °C, 18,5 Teile halogeniertes 1,1,4,4-Tetramethylbutan-1,4-diisocyanat (je Molekül 3 Chloratome),
b) Kp (0,133 mbar) 130 bis 160 °C, 21 Teile halogeniertes 1,1,4,4-Tetramethylbutan-1,4-diisocyanat (je Molekül 6 Chloratome),
c) Kp (0,133 mbar) 167 bis 190 °C, 40 Teile halogeniertes 1,1,4,4-Tetramethylbutan-1,4-diisocyanat (je Molekül 10 Chloratome).

Beispiel 3

In 120 Teile tert.-Butylisocyanat leitet man bei 10 °C elementares Chlor unter Belichtung mit einer Leuchtstoffröhre (4 000 bis 7 000 Angström) ein. Während des Einleitens steigert man langsam die Reaktionstemperatur während 4 Std. auf 160 °C. Nach einer Absorption von 90 Teilen Chlorgas bläst man die Lösung mit trockenem Stickstoff durch und destilliert das Gemisch fraktioniert im Vakuum. Man erhält folgende Fraktionen:

d) Kp (24 mbar) 86 bis 118 °C 25 Teile halogeniertes tert.-Butylisocyanat (3 Chloratome),
e) Kp (0,27 mbar) 64 bis 74 °C 70 Teile halogeniertes tert.-Butylisocyanat (5 Chloratome),
f) Kp (0,27 mbar) 86 bis 97 °C 96 Teile (Gemisch von 80 Gew.-% 1-(Trichlormethyl)-1-(dichlormethyl)-2-chloräthylisocyanat und 20 Gew.-% 1,1-Bis(dichlormethyl)-2,2-dichloräthylisocanat.

Eine Recyclisierung niederer chlorierter Anteile zur Erzielung möglichst hoher Anteile mit hohem Chlorgehalt ist vorteilhaft.

Beispiel 4

Anstelle von tert.-Butylisocyanat werden 164 Teile rohes tert.-Butylcarbamoylchlorid verwendet, wie es bei der Phosgenierung von tert.-Butylamin anfällt. Man führt die Umsetzung analog Beispiel 3 durch, wobei man die Chlorierung bei 80 °C beginnt. Man erhält folgende Fraktionen:

g) Kp (24 mbar) 86 bis 118 °C 25 Teile halogeniertes tert.-Butylisocyanat (3 Chloratome),
h) Kp (0,27 mbar) 64 bis 74 °C 70 Teile halogeniertes tert.-Butylisocyanat (5 Chloratome),
i) Kp (0,27 mbar) 86 bis 97 °C 96 Teile Gemisch von 80 Gew.-% 1-(Trichlormethyl)-1-(dichlormethyl)-2-chloräthylisocyanat und 20 Gew.-% 1,1-Bis(dichlormethyl)-2,2-dichloräthylisocyanat.

Beispiel 5

Zu 844 Teilen t-Butylisocyanat fügt man 951 Teile der niederhalogenierten Fraktionen d und e (gemäss Beispiel 3) hinzu, leitet unter Belichtung mit einer UV-Tauchlampe bei zunächst 30 bis 40 °C Chlor ein. Die exotherme Reaktion lässt man – unter weiterem Chloreinleiten – bis auf 100 °C ansteigen. Mit steigender Chloraufnahme muss man schliesslich zusätzlich erhitzen (bis 135 °C). Nach einer Gewichtszunahme von 1714 Teilen (= 24 Mol) wird überschüssiger gelöster Chlorwasserstoff mit trockenem Stickstoff bei 120 °C ausgeblasen und dann im Vakuum fraktioniert destilliert. Man erhält – bei Destillation über eine 30 cm Füllkörperkolonne folgende Fraktionen:

1. $Kp_{(0,1\ mbar)}$ 45–49°–190 Teile $n_D^{25}$ = 1,5096
2. $Kp_{(0,1\ mbar)}$ 49–54°–163 Teile $n_D^{25}$ = 1,5180

3. $Kp_{(0,1\ mbar)}$ 54–55°–185 Teile $n_D^{25}$ = 1,5209
4. $Kp_{(0,1\ mbar)}$ 55–57°–190 Teile $n_D^{25}$ = 1,5220
5. $Kp_{(0,1\ mbar)}$ 57° –183 Teile $n_D^{25}$ = 1,5230
6. $Kp_{(0,1\ mbar)}$ 57–60°–181 Teile $n_D^{25}$ = 1,5243
7. $Kp_{(0,1\ mbar)}$ 60–62°–167 Teile $n_D^{25}$ = 1,5258
8. $Kp_{(0,1\ mbar)}$ 62–66°–245 Teile $n_D^{25}$ = 1,5279
9. $Kp_{(0,1\ mbar)}$ 66–85°–447 Teile $n_D^{25}$ = kristallin
10. $Kp_{(0,1\ mbar)}$ 85–88°–390 Teile $n_D^{25}$ = kristallin

Destillationsrückstand: ca. 75 Teile.

Alle Fraktionen ab 5. Fraktion (incl.) enthalten Isomerengemische mit 6, Fraktion 9 und 10 geringe Anteile mit 7 Chloratomen pro Mol tert.-Butylisocyanat (67,5 bis 70,9% Chlor). Hauptkomponente in der Fraktion 10 ist: (1-(Trichlormethyl)-1-(dichlormethyl)-2-chlorethylisocyanat.

Die Fraktionen 1 bis 4 können erneut zur Chlorierung eingesetzt werden.

Beispiel 6

47 Teile 1,1,2-Trimethylpropylisocyanat werden mit einer aussen am Kolben befestigten Laborlampe – beginnend bei 0° – chloriert. Die exotherme Reaktion steigt bis ca. 120° an. Die Gesamtgewichtszunahme – nach Reaktionsführung bei 130° – beträgt 66 Teile (= 0,93 Mol). Man fraktioniert – nach Ausblasen des gelösten Chlorwasserstoffes – im Ölpumpenvakuum:

| | O | N | Cl |
|---|---|---|---|
| $Kp_{(0,1\ mbar)}$ 49– 50°– 9 Teile $n_D^{20}$ × 1,5276 | 5,1 | 4,3 | 61,7 |
| $Kp_{(0,1\ mbar)}$ 102–110°–16 Teile $n_D^{20}$ = 1,5454 | 4,9 | 4,4 | 61,9 |
| $Kp_{(0,1\ mbar)}$ 110– 5°–22 Teile $n_D^{20}$ = 1,5461 | 4,8 | 4,3 | 63,0 |
| $Kp_{(0,1\ mbar)}$ 115– 7°–23 Teile $n_D^{20}$ = 1,5491 | 4,7 | 4,0 | 64,6 |
| $Kp_{(0,1\ mbar)}$ 117– 25°–18 Teile $n_D^{20}$ = 1,5571 | 4,8 | 4,2 | 63,8 |

5 Teile Destillationsrückstand.

Berechnet für $C_7H_7ONCl_6$ O = 4,8 N = 4,2 Chlor 63,8.

Beispiel 7

In analoger Weise – wie im Beispiel 5 – werden 120 Teile 1,5-Dimethyl-1-ethyl-hexylisocyanat chloriert. Von dem farblosen, viskosen Öl ($n_D^{22}$ = 1,5320) werden 90 Teile fraktioniert destilliert:

$Kp_{(0,2\ mbar)}$ 36–165°– 3 Teile $n_D^{22}$ = 1,5195
$Kp_{(2,5\ mbar)}$ 165–175°– 7 Teile $n_D^{22}$ = 1,5215
$Kp_{(1,0\ mbar)}$ 175– 80°–15 Teile $n_D^{22}$ = 1,5255
$Kp_{(2,0\ mbar)}$ 180–184°–20 Teile $n_D^{22}$ = 1,5295
$Kp_{(2,0\ mbar)}$ 184–187°–17 Teile $n_D^{22}$ = 1,5368

Nach den O, N, Chlor-Analysen enthalten die Fraktionen 2 und 3 vornehmlich 5, die Fraktionen 4 und 5 besonders 6 Chloratome pro Isocyanatmolekül.

Beispiel 8

(Chloranlagerung mit anschliessender Substitution)

Unter Kühlung (Eisbad) lagert man zunächst bei ca. 20 °C 82 Teile elementares Chlor an 64 Teile 1,2-Dimethyl-butin-(2)yl-isocyanat an. Das farblose 1,2-Dimethyl-3,4-tetrachlorbutyl(2)-isocyanat destilliert bei $Kp_{0,1\ mbar}$ 63–64 °C ($n_D^{22}$ = 1,5070).

Dieses wird – wie im Beispiel 6 aufgezeigt – mit elementarem Chlor bei 80 bis 120 °C weiter chloriert (bis 39 Teile Gewichtszunahme). Bei der fraktionierten Destillation (ohne Kolonne) ergaben sich – nach einem kurzen Vorlauf – folgende Fraktionen:

$Kp_{(0,1\ mbar)}$ 62– 70°–16 Teile $n_D^{24}$ = 1,5240
$Kp_{(0,1\ mbar)}$ 75–100°–12 Teile $n_D^{24}$ = 1,5450
$Kp_{(0,1\ mbar)}$ 103–115°–63 Teile $n_D^{24}$ = 1,5540
$Kp_{(0,1\ mbar)}$ 115–129°–28 Teile $n_D^{24}$ = 1,5600

Die Hauptfraktionen 4 und 5 sind – nach NMR sowie Isocyanatgruppen – und O, N, Chlorbestimmung – 7 Chloratome enthaltende Stoffe mit dem Hauptprodukt: 1-Dichlormethyl-2-chlormethyl-3,4-(tetrachlor)-butyl(2)-isocyanat.

Beispiel 9

Zu 100 Teilen (0,67 Mol) 1-(Ethinyl)-cyclohexylisocyanat (1) werden bei 210° 214 Teile (1,34 Mol) Brom zugetropft. Das resultierende 1-Tetrabromethyl)-cyclohexylisocyanat (1) – vom $Kp_{0,2\ mbar}$ 118 bis 22°, $n_D^{23}$ = 1,5611 – dient als Ausgangsprodukt für nachfolgendes gemischt-halogeniertes Isocyanat:

90 Teile vorstehenden 1-(Tetrabromethyl)-cyclohexylisocyanates (1) werden – gelöst in 180 Teilen Tetrachlorethan – unter Belichtung mit UV-Licht zunächst bei 50 bis 90° chloriert und schliesslich – bis zur Aufnahme von 26 Teilen Chlor – bis zum Sieden erhitzt. Die Lösung wird noch heiss mit trockenem Stickstoff ausgeblasen, wobei gelbbraune Dämpfe entweichen. Dann wird das Lösungsmittel im Vakuum abgezogen und schliesslich fraktioniert destilliert.

$Kp_{(0,2\ mbar)}$ 108–135° (Vorlauf) – 4 Teile $n_D^{20}$ = 1,5729
$Kp_{(0,2-0,5\ mbar)}$ 135–146° (Vorlauf) – 12 Teile $n_D^{20}$ = 1,5750
$Kp_{(0,5\ mbar)}$ 144–146° (Vorlauf) – 54 Teile $n_D^{20}$ = 1,5772

Es verbleiben 3 Teile Rückstand.

Die Hauptfraktion zeigt folgende Analysenwerte: O = 4,2% N = 3,2% Br = 31,4% Cl = 28,7%.

**Patentansprüche**

1. Verfahren zur Herstellung von halogenierten, tertiären Alkylisocyanaten der Formel

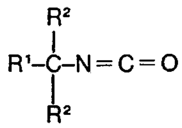 I,

worin $R^1$ den Rest $R^2$ oder den Rest

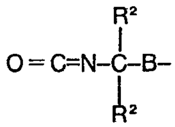

bedeutet, die einzelnen Reste $R^2$ gleich oder verschieden sind und jeweils einen halogenierten oder halogenfreien Alkylrest mit 1 bis 6 Kohlenstoffatomen bedeuten, jeweils zwei über ein Kohlenstoffatom benachbarte Reste $R^2$ auch Glieder eines halogenfreien oder halogenierten, alicyclischen, 5- oder 6gliedrigen Ringes bilden können, B einen halogenfreien oder halogenierten Alkylenrest mit 1 bis 6 Kohlenstoffatomen bezeichnet, mindestens einer der Reste $R^2$ ein oder mehrere Halogenatome trägt, wobei die Gesamtzahl der Halogenatome mindestens 3 und, wenn $R^1$ den Rest $R^2$ bezeichnet und alle 3 Reste $R^2$ zusammen 3 Kohlenstoffatome enthalten, mindestens 4 beträgt, alle Reste $R^2$ und gegebenenfalls B zusammen höchstens 9 Kohlenstoffatome bezeichnen, durch Halogenierung von Isocyanaten oder Carbamidsäurechloriden, dadurch gekennzeichnet, dass man tertiäre Alkylisocyanate der Formel

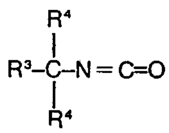 II,

oder tertiäre Carbamidsäurehalogenide der Formel

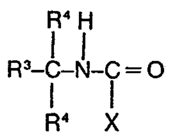 III,

worin $R^3$ den Rest $R^4$ oder den Rest

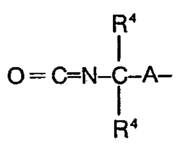

oder

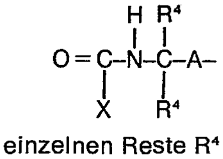

bedeutet, die einzelnen Reste $R^4$ gleich oder verschieden sein können und jeweils einen halogenierten oder halogenfreien Alkylrest mit 1 bis 6 Kohlenstoffatomen bedeuten, jeweils 2 über ein Kohlenstoffatom benachbarte Reste $R^4$ auch Glieder eines halogenfreien oder halogenierten, alicyclischen, 5- oder 6gliedrigen Ringes bilden können, A einen Alkylenrest oder Halogenalkylenrest mit 1 bis 6 Kohlenstoffatomen bezeichnet, alle Reste $R^4$ und gegebenenfalls A zusammen höchstens 9 Kohlenstoffatome bezeichnen, X für ein Halogenatom steht, mit Halogen, Sulfurylchlorid und/oder Sulfurylbromid umsetzt.

2. Halogenierte, tertiäre Alkylisocyanate der Formel

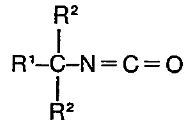 I,

worin $R^1$ den Rest $R^2$ oder den Rest

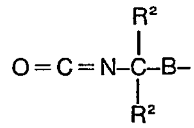

bedeutet, die einzelnen Reste $R^2$ gleich oder verschieden sind und jeweils einen halogenierten oder halogenfreien Alkylrest mit 1 bis 6 Kohlenstoffatomen bedeuten, jeweils zwei über ein Kohlenstoffatom benachbarte Reste $R^2$ auch Glieder eines halogenfreien oder halogenierten, alicyclischen, 5- oder 6gliedrigen Ringes bilden können, B einen halogenfreien oder halogenierten Alkylenrest mit 1 bis 6 Kohlenstoffatomen bezeichnet, mindestens einer der Reste $R^2$ ein oder mehrere Halogenatome trägt, wobei die Gesamtzahl der Halogenatome mindestens 3 und, wenn $R^1$ den Rest $R^2$ bezeichnet und alle 3 Reste $R^2$ zusammen 3 Kohlenstoffatome enthalten, mindestens 4 beträgt, alle Reste $R^2$ und gegebenenfalls B zusammen höchstens 9 Kohlenstoffatome bezeichnen.

**Claims**

1. A process for the preparation of halogenated tertiary alkyl isocyanates of the formula

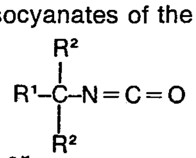 I,

where $R^1$ is $R^2$ or

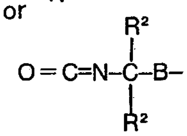 ,

and the individual radicals $R^2$ are identical or different and each is haloalkyl or halogen-free alkyl of 1 to 6 carbon atoms, and two radicals $R^2$ bonded to the same carbon atom may also be members of a halogen-free or halogenated alicyclic 5- or 6-membered ring, B is halogen-free alkylene or haloalkylene of 1 to 6 carbon atoms, and one or more of the radicals $R^2$ possess one or more halogen atoms, and the total number of halogen atoms is at least 3 and, if $R^1$ is $R^2$ and all three radicals $R^2$ together contain 3 carbon atoms, is at least 4, and all radicals $R^2$ together, with or without B, have not more than 9 carbon atoms, by halogenation of isocyanates or carbamic acid chlorides, wherein a tertiary alkyl isocyanate of the formula

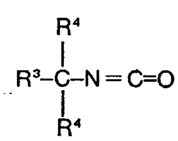 II,

or a tertiary carbamic acid halide of the formula

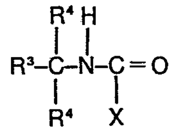 III,

where $R^3$ is $R^4$ or

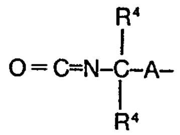

or

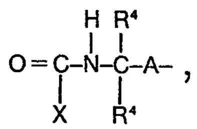

the individual radicals $R^4$ may be identical or different and each is haloalkyl or halogen-free alkyl of 1 to 6 carbon atoms, and two radicals $R^4$ bonded to the same carbon atom may also be members of a halogen-free or halogenated alicyclic 5- or 6-membered ring, A is alkylene or haloalkylene of 1 to 6 carbon atoms, all radicals $R^4$ together, with or without A, have not more than 9 carbon atoms, and X is halogen, is reacted with halogen, sulfuryl chloride and/or sulfuryl bromide.

2. A halogenated tertiary alkyl isocyanate of the formula

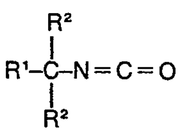 I,

where $R^1$ is $R^2$ or

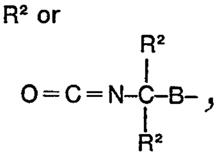

and the individual radicals $R^2$ are identical or different and each is haloalkyl or halogen-free alkyl of 1 to 6 carbon atoms, and two radicals $R^2$ bonded to the same carbon atom may also be members of a halogen-free or halogenated alicyclic 5- or 6-membered ring, B is halogen-free alkylene or haloalkylene of 1 to 6 carbon atoms, and one or more of the radicals $R^2$ possess one or more halogen atoms, and the total number of halogen atoms is at least 3 and, if $R^1$ is $R^2$ and all 3 radicals $R^2$ together contain 3 carbon atoms, is at least 4, and all radicals $R^2$ together, with or without B, have not more than 9 carbon atoms.

**Revendications**

1. Procédé de préparation d'isocyanates d'alcoyle tertiaires halogénés de la formule

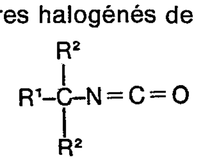 I,

dans laquelle $R^1$ possède la signification de $R^2$ ou désigne un groupe

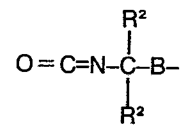

et les divers restes $R^2$, qui peuvent être identiques ou différents, représentent chacun un radical alcoyle halogéné ou exempt d'halogène en $C_1$ à $C_6$, deux restes $R^2$, reliés par un atome de carbone, pouvant en outre constituer des chaînons d'un noyau alicyclique penta- ou hexagonal halogéné ou non et B représente un groupe alcoylène halogéné ou non en $C_1$ à $C_6$, au moins un des restes $R^2$ portant un ou plusieurs atomes d'halogène, le nombre total des atomes d'halogène étant de 3 au moins et de 4 au moins, lorsque $R^1 = R^2$ et les trois restes $R^2$ contiennent ensemble trois atomes de carbone, et tous les restes $R^2$ et éventuellement B comportant ensemble au maximum 9 atomes de carbone, par halogénation d'isocyanates ou de chlorures d'acide carbamique, caractérisé en ce que l'on fait réagir des isocyanates d'alcoyle tertiaires de la formule

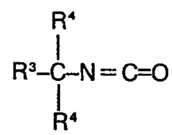 II,

ou des halogénures d'acide carbamique tertiaires de la formule

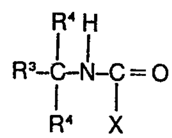 III,

dans lesquelles $R^3$ possède la signification de $R^4$ ou désigne un groupe

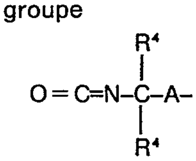

ou

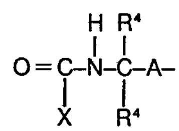

et les divers restes $R^4$, qui peuvent être identiques ou différents, représentent chacun un radical alcoyle halogéné ou exempt d'halogène en $C_1$ à $C_6$, deux restes $R^4$, reliés par un atome de carbone, pouvant en outre constituer des chaînons d'un noyau alicyclique penta- ou hexagonal halogéné ou non et A représente un groupe alcoylène ou haloalcoylène en $C_1$ à $C_6$, tous les restes $R^4$ et éventuellement A comportant ensemble au maximum 9 atomes de carbone, et X désigne un atome d'halogène, avec un halogène, le chlorure de sulfuryle et(ou) le bromure de sulfuryle.

2. Isocyanates d'alcoyle tertiaires halogénés de la formule

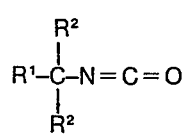 I,

dans laquelle $R^1$ possède la signification de $R^2$ ou désigne un groupe

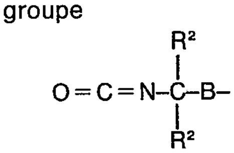

et les divers restes $R^2$, qui peuvent être identiques ou différents, représentant chacun un radical alcoyle en $C_1$ à $C_6$ halogéné ou non halogéné, deux restes $R^2$, reliés par un atome de carbone, pouvant en outre constituer des chaînons d'un noyau alicyclique penta- ou hexagonal halogéné ou non et B représente un groupe alcoylène halogéné ou non en $C_1$ à $C_6$, au moins un des restes $R^2$ portant un ou plusieurs atomes d'halogène, le nombre total des atomes d'halogène étant de 3 au moins et de 4 au moins, lorsque $R^1$ = $R^2$ et les trois restes $R^2$ contiennent ensemble trois atomes de carbone, le nombre total des atomes de carbone de tous les restes $R^2$ et éventuellement de B ensemble étant au maximum de 9.